# FASCICULE DE BREVET EUROPEEN

(11) **EP 1 276 870 B1**
(45) Date de publication et mention de la délivrance du brevet: **06.06.2007**
(21) Numéro de dépôt: 01929693.8
(22) Date de dépôt: 24.04.2001
(51) Int. Cl.: C12N 15/29, C12N 15/82, C12Q 1/68, A01H 5/00

(54) **PROCEDE D'OBTENTION DE PLANTES PRESENTANT UNE RESISTANCE ACCRUE A UN STRESS HYDRIQUE**
VERFAHREN ZUR HERSTELLUNG VON PFLANZEN MIT ERHÖHTER RESISTENZ GEGEN WASSERSTRESS
METHOD FOR OBTAINING PLANTS EXHIBITING ENHANCED RESISTANCE TO WATER STRESS

(30) Priorité: 28.04.2000 FR 0005534
(43) Date de publication de la demande: 22.01.2003
(73) Titulaire: Biogemma, 75001 Paris (FR); INSTITUT NATIONAL DE LA RECHERCHE AGRONOMIQUE (INRA), 75338 Paris Cédex 07 (FR); ARVALIS - INSTITUT DU VEGETAL, 75116 Paris (FR)
(72) Inventeur: ZIVY, Michel, F-75010 Paris (FR); PEREZ, Pascual, F-63450 Chanonat (FR)
(74) Mandataire: Bernasconi, Jean Raymond
(86) Numéro de dépôt international: PCT/FR2001/001252
(87) Numéro de publication internationale: WO 2001/083756

(56) Documents cités:
- WO-A-97/13843
- RICCARDI FREDERIQUE ET AL: "Protein changes in response to progressive water deficit in maize." PLANT PHYSIOLOGY (ROCKVILLE), vol. 117, no. 4, août 1998 (1998-08), pages 1253-1263, XP002157440 ISSN: 0032-0889
- ROSSI MAGDALENA ET AL: "Asr genes belong to a gene family comprising at least three closely linked loci on chromosome 4 in tomato." MOLECULAR & GENERAL GENETICS, vol. 252, no. 4, 1996, pages 489-492, XP002157441 ISSN: 0026-8925
- SILHAVY DANIEL ET AL: "Isolation and characterization of a water-stress-inducible cDNA clone from Solanum chacoense." PLANT MOLECULAR BIOLOGY, vol. 27, no. 3, 1995, pages 587-595, XP002157442 ISSN: 0167-4412
- XU D ET AL: "EXPRESSION OF A LATE EMBRYOGENESIS ABUNDANT PROTEIN GENE, HVA1, FROM BARLEY CONFERS TOLERANCE TO WATER DEFICIT AND SALT STRESS IN TRANSGENIC RICE" PLANT PHYSIOLOGY,US,AMERICAN SOCIETY OF PLANT PHYSIOLOGISTS, ROCKVILLE, MD, vol. 110, no. 1, 1996, pages 249-257, XP002034436 ISSN: 0032-0889
- DATABASE EMBL [en ligne] ACCESSION NO: AW455731, 24 février 2000 (2000-02-24) WALBOT, V: "707090G04.x1 707 - Mixed adult tissues from Walbot lab (SK) Zea mays cDNA,mRNA sequence." XP002157443
- DATABASE EMBL [en ligne] ACCESSION NO: AI677361, 26 mai 1999 (1999-05-26) WALBOT, V.: "605054A04.x1 605 - Endosperm cDNA library from Schmidt lab Zea mays cDNA,mRNA sequence." XP002157444
- DATABASE EMBL [en ligne] ACCESSION NO: AA979957, 27 mai 1998 (1998-05-27) WEN T.J.: "MEST4-B11.TW1412.Seq ISUM2 Zea mays cDNA clone MEST4-B11 5', mRNA sequence." XP002157445
- DATABASE EMBL [en ligne] ACCESSION NO: U09276, 3 octobre 1994 (1994-10-03) ARREDONDO-PETER R., ET AL.: "Zea mays ABA- and ripening-inducible-like protein mRNA, complete cds." XP002157446
- DATABASE EMBL [en ligne] ACCESSION NO: AI966921, 24 août 1999 (1999-08-24) WALBOT, V.: "496019C12.x1 496 - stressed shoot cDNA library from Wang/Bohnert lab Zea mays cDNA, mRNA sequence." XP002157447
- DATABASE EMBL [en ligne] ACCESSION NO: AF039573, 17 janvier 1998 (1998-01-17) VAIDYANATHAN R., ET AL.: "Oryza sativa abscisic acid- and stress-inducible protein (Asr1) mRNA," XP002157448

### LISTE DE SEQUENCES

<110> BIOGEMMA
<120> Procédé d'obtention de plantes présentant une résistance accrue à un stress hydrique
<130> BFF 00/0279
<140>
   <141>
<160> 7
<170> PatentIn Ver. 2.1
<210> 1
   <211> 777
   <212> ADN
   <213> Zea mays
<220>
   <221> CDS
   <222> (85)..(504)
<400> 1
   tgtcgatcca attgtcactt gctctccctc caacaagcta attaaggccg gtcgtcatcc 60
   ctcttctagc tcgttttatt atcc atg gcg gag gag aag cac cac cac cac    111
                              Met Ala Glu Glu Lys His His His His
                               1               5
   cac ctg ttc cac cat aag aag gac gag gag cag gag gag cag ctc gcc   159
   His Leu Phe His His Lys Lys Asp Glu Glu Gln Glu Glu Gln Leu Ala
    10                  15                  20                  25
   ggc ggc ggg tac ggc gag tcc gcc gag tac acg gag gcc acg gtg acg   207
   Gly Gly Gly Tyr Gly Glu Ser Ala Glu Tyr Thr Glu Ala Thr Val Thr
                    30                  35                  40
   gag gtg gtg tcc acg ggc gag aac gag tac gac gag tac aag gag gag   255
   Glu Val Val Ser Thr Gly Glu Asn Glu Tyr Asp Glu Tyr Lys Glu Glu
                45                  50                  55
   aag cag cat aag cac aag cag cac ctc ggc gag gcc ggc gcc atc gcc   303
   Lys Gln His Lys His Lys Gln His Leu Gly Glu Ala Gly Ala Ile Ala
            60                  65                  70
   gcc ggc gcc ttc gca ctc tac gag aag cac gag gca aag aag gac ccg   351
   Ala Gly Ala Phe Ala Leu Tyr Glu Lys His Glu Ala Lys Lys Asp Pro
        75                  80                  85
   gag cac gcg cac cgc cac aag atc gag gag gag gtc gcg gcg gcg gcg   399
   Glu His Ala His Arg His Lys Ile Glu Glu Glu Val Ala Ala Ala Ala
    90                  95                 100                 105
   gcc gtc ggc tcc ggc ggc ttc gcc ttc cac gag cac cac gag aag aag   447
   Ala Val Gly Ser Gly Gly Phe Ala Phe His Glu His His Glu Lys Lys
                   110                 115                 120
   aag gac cac aag gac gcc gag gag gcc ggc ggc gag aag aag cac cac   495
   Lys Asp His Lys Asp Ala Glu Glu Ala Gly Gly Glu Lys Lys His His
               125                 130                 135
   ttc ttc ggc tgattgatcc ctcccgtatc gtcgtccctc cccgtgtgct            544
   Phe Phe Gly
           140
   acgcgtgcgt gtgtgagagt gatatcgagc gcccgccgtg ttgtgcgcgc gtacgtatgt 604
   atgcgctcgt gtgatgcacg aataagcgtg gctacgtaat ctatcgtatg tatacgtgtg 664
   tgtatgcatg tgcttgtgta tgatcgtggt acgaggaccg aaaaaatgta tgcaactctg 724
   atttacttac atgtttagtt gtttacgttt ctccaaaaaa aaaaaaaaaa aaa        777
<210> 2
   <211> 140
   <212> PRT
   <213> Zea mays
<400> 2
   Met Ala Glu Glu Lys His His His His His Leu Phe His His Lys Lys
   1 5 10 15
   Asp Glu Glu Gln Glu Glu Gln Leu Ala Gly Gly Gly Tyr Gly Glu Ser
   20 25 30
   Ala Glu Tyr Thr Glu Ala Thr Val Thr Glu Val Val Ser Thr Gly Glu
   35 40 45
   Asn Glu Tyr Asp Glu Tyr Lys Glu Glu Lys Gln His Lys His Lys Gln
   50 55 60
   His Leu Gly Glu Ala Gly Ala Ile Ala Ala Gly Ala Phe Ala Leu Tyr
   65 70 75 80
   Glu Lys His Glu Ala Lys Lys Asp Pro Glu His Ala His Arg His Lys
   85 90 95
   Ile Glu Glu Glu Val Ala Ala Ala Ala Ala Val Gly Ser Gly Gly Phe
   100 105 110
   Ala Phe His Glu His His Glu Lys Lys Lys Asp His Lys Asp Ala Glu
   115 120 125
   Glu Ala Gly Gly Glu Lys Lys His His Phe Phe Gly
   130 135 140
<210> 3
   <211> 892
   <212> ADN
   <213> Zea mays
<400> 3
   tgtcgatcca attgtcactt gctctccctc caacaagcta attaaggccg gtcgtcatcc 60
   ctcttctagc tcgttttatt atccatggcg gaggagaagc accaccacca ccacctgttc 120
   caccataaga aggacgagga gcaggaggag cagctcgccg gcggcgggta cggcgagtcc 180
   gccgagtaca cggaggccac ggtgacggag gtggtgtcca cgggcgagaa cgagtacgac 240
   gagtacaagg aggagaagca gcataagcac aagcagcacc tcggcgaggc cggcgccatc 300
   gccgccggcg ccttcgcact cgtacgtagt cctccgatcg atccgatcct ccttgagtag 360
   tatatacata catgaacgcg ataacgaata atatattaat cgaacgaact gaatgatgat 420
   cacggatcac ctcgtgtgac gtggacatgc acagtacgag aagcacgagg caaagaagga 480
   cccggagcac gcgcaccgcc acaagatcga ggaggaggtc gcggcggcgg cggccgtcgg 540
   ctccggcggc ttcgccttcc acgagcacca cgagaagaag aaggaccaca aggacgccga 600
   ggaggccggc ggcgagaaga agcaccactt cttcggctga ttgatccctc ccgtatcgtc 660
   gtccctcccc gtgtgctacg cgtgcgtgtg tgagagtgat atcgagcgcc cgccgtgttg 720
   tgcgcgcgta cgtatgtatg cgctcgtgtg atgcacgaat aagcgtggct acgtaatcta 780
   tcgtatgtat acgtgtgtgt atgcatgtgc ttgtgtatga tcgtggtacg aggaccgaaa 840
   aaatgtatgc aactctgatt tacttacatg tttagttgtt tacgtttctc ca 892
<210> 4
   <211> 890
   <212> ADN
   <213> Zea mays
<400> 4
   tgtcgatcca attgtcactt gctctccctc caacaagcta attaaggccg gtcatccctc 60
   ttctagctcg tttcattatc catggcggag gagaagcacc accaccacca cctgttccac 120
   cacaagaagg acgaggagca ggaggagcag ctcgccggcg gcgggtacgg cgagtccgcc 180
   gagtacacgg aggccacggt gacggaggtg gtgtccacgg gcgagaacga gtacgacgag 240
   tacaagaagg aggagaagca gcacaagcac aagcagcacc tcggcgaggc cggcgccatc 300
   gccgccggcg ccttcgcact cgtacgtagt cctccgatcg atccgatcct ccttgagtag 360
   tatatacata catgaacgcg ataacgaata atatattaat cgaacgaact gaatgatgat 420
   cacggatcac ctcgtgtgac gtggacatgc acagtacgag aagcacgagg caaagaagga 480
   cccggagcac gcgcaccgcc acaagatcga ggaggaggtc gcggcggcgg cggccgtcgg 540
   ctccggcggc ttcgccttcc acgagcacca cgagaagaag aaggaccaca aggacgccga 600
   ggaggccggc ggcgagaaga agcaccactt cttcggctga ttgatccctc ccgtatcgtc 660
   gtccctcccc gtgtactacg cgtgcgtgtg tgagagtgat atcgagcgcc cgccgtgttg 720
   tgcgcgcgta cgtatgtatg cgctcgtgtg atgcacgaat aagcgtggct acgtaatcta 780
   tcgtatgtat acgtgtgtgt atgcatgtgc ttgtgtatga tcgtggtacg aggaccgaaa 840
   aaatgtatgc aactctgatt tacttacatg tttagttgtt tacgtttctc           890
<210> 5
   <211> 814
   <212> ADN
   <213> Zea mays
<400> 5
   gctaattaag gccggtcgtc atccctcttc tagctcgttt tattatccat ggcggaggag 60
   aagcaccacc accaccacct gttccaccat aagaaggacg aggagcagga ggagcagctc 120
   gccggcggcg ggtacggcga gtccgccgag tacacggagg ccacggtgac ggaggtggtg 180
   tccacgggcg agaacgagta cgacgagtac aagaaggagg agaagcagca caagcacaag 240
   cagcacctcg gcgaggccgg cgccatcgcc gccggcgcct tcgcactcgt acgtagtcct 300
   ccgatcgatc cgatcctcct tgagtagtat atacatacat gaacgcgata acgaataata 360
   tattaatcga acgaactgaa tgatgatcac ggatcacctc gtgtgacgtg gacatgcaca 420
   gtacgagaag cacgaggcaa agaaggaccc ggagcacgcg caccgccaca agatcgagga 480
   ggaggtcgcg gcggcggcgg ccgtcggctc cggcggcttc gccttccacg agcaccacga 540
   gaagaagaag gaccacaagg acgccgagga ggccggcggc gagaagaagc accacttctt 600
   cggctgattg atcctcccgt atcgtcgtcc ctccccgtgt gctacgcgtg cgtgtgtgag 660
   actgatatcg agcgcccgcc gtgttgtgcg cgcgtacgta tgtatgcgct cgtgtgatgc 720
   acgaataagc gtggctacgt aatctatcgt atgtatacgt gtgtgtatgc atgtgcttgt 780
   gtatgatcgt ggnacgagga ccgaaaaaat gtat                             814
<210> 6
   <211> 20
   <212> ADN
   <213> Zea mays
<400> 6
   tgtcgatcca attgtcactt 20
<210> 7
   <211> 20
   <212> ADN
   <213> Zea mays
<400> 7
   tggagaaacg taaacaacta 20

## Description

La présente invention concerne un procédé d'obtention de plantes présentant une résistance accrue à un stress hydrique.

Dans les régions tempérées, des périodes de faible pluviométrie, d'intensités variables et imprévisibles sont cause d'une baisse de productivité notable chez les plantes en culture. Le déficit hydrique peut affecter sévèrement la croissance et la reproduction des plantes. Différentes stratégies faisant appel à des mécanismes physiologiques (réduction de la croissance des parties aériennes, fermeture des stomates) et/ou cellulaires (ajustement osmotique) peuvent leur permettre d'échapper à ce stress hydrique, tout au moins de le tolérer. Ces mécanismes, au moins en partie contrôlés par l'ABA (acide abscissique), phytohormone dont la concentration augmente dans les plantes soumises à un stress hydrique (Zeevaart and Creelman, 1988), impliquent de nombreuses protéines de fonctions putatives différentes : protéines de type canaux membranaires, ou exprimées en réponse à des dommages causés à la cellule (protéases, inhibiteurs de protéases) ou encore des protéines dont les fonctions ne sont pas directement liées au stress mais qui sont exprimées à des niveaux plus élevés en condition de stress hydrique ou salin (enzymes de la glycolyse, de la synthèse de la méthionine). La réponse du végétal dépend par ailleurs de paramètres environnementaux (type de contrainte, intensité, durée) et génétiques (espèce et génotype), rendant difficile la détermination du rôle de ces protéines dans les mécanismes de tolérance à la sécheresse.

Il existe donc un réel besoin de mettre en évidence la fonction de gènes candidats dans les mécanismes de tolérance au stress hydrique pour leur utilisation en transgénèse visant l'obtention de plantes présentant une meilleure tolérance au stress hydrique. Ceci est particulièrement important pour les espèces de grande culture, comme le maïs par exemple, qui atteint un niveau de sensibilité maximale à la sécheresse 15 jours avant et 15 jours après la floraison de la plante (juillet-août en Europe), période pendant laquelle la plante absorberait 45% du total de ses besoins.

Une première étude réalisée par Riccardi et al. (1998) sur une lignée de maïs désignée Io (Iodent) a mis en évidence une vingtaine de protéines dont l'expression est significativement augmentée en réponse à un stress hydrique, et pour lesquelles des fonctions putatives ont été proposées.

Une de ces protéines, mise en évidence dans la lignée Io utilisée par Riccardi et al, (1998) mais pas dans la lignée F2, présente des homologies avec une protéine ASR1 ("ABA-water stress-ripening-induced protein") de tomate, qui est induite par l'ABA, le stress hydrique et le mûrissement du fruit, mais dont la fonction est encore inconnue (Iusem et al., 1993). D'autres gènes montrant des homologies avec l'ASR de tomate ont été identifiés notamment chez la pomme de terre (Silhavy et al., 1995), le citron (Canel et al., 1995), le riz (Thomas et al., 1999) mais aucune fonction n'a été clairement démontrée pour les protéines codées par ces gènes. Une étude QTL ("Quantitative Trait loci") a permis de cartographier le gène Asr1 dans une région contenant à la fois le locus contrôlant la quantité d'ASR1 et des QTLs de sénescence foliaire et protandrie (De Vienne et al., 1999).

Les auteurs de la présente invention ont maintenant mis en évidence le rôle d'une protéine ASR recombinante dans la réponse directe d'une plante à un stress hydrique pour l'utilisation de la transgénèse. En effet, ils sont parvenus à mettre au point un procédé d'obtention d'une plante présentant une quantité modifiée de protéine ASR, lui conférant une meilleure résistance au stress hydrique par rapport à une plante non transformée.

L'invention a donc pour objet un procédé d'obtention d'une plante contenant une quantité modifiée de protéine ASR, lui conférant une meilleure résistance au stress hydrique par rapport à une plante non transformée, comprenant les étapes consistant à :
- transformer au moins une cellule de plante avec un vecteur, contenant une cassette d'expression comprenant une séquence nucléotidique codant pour une protéine ASR;
- cultiver la cellule ainsi transformée de manière à générer une plante contenant dans son génome ladite cassette d'expression.

Par "protéine ASR", on entend une protéine exprimée naturellement par une plante en réponse à un stress hydrique, ayant une séquence d'acides aminés identique ou homologue à la séquence SEQ ID n° 2 de maïs.

Par "séquence homologue", on entend de préférence une séquence présentant au moins 50 %, de préférence 70 % de similitude avec la séquence SEQ ID n° 2.

Sont comprises dans la définition de "protéine ASR" au sens de l'invention, toutes les protéines ASR de divers végétaux, comme par exemple celle du riz, ainsi que les protéines modifiées, par exemple par addition, délétion, substitution, (de préférence conservative), d'un nombre réduit d'acides aminés.

Sont également compris dans la définition de "protéine ASR", les polypeptides codés par tout ou partie de la séquence SEQ ID n° 1, n° 3, n° 4 ou n° 5, ou de toute séquence homologue, étant entendu que ces polypeptides conservent la propriété de résistance à un stress hydrique.

La séquence d'acide nucléique codant pour une protéine ASR peut être plus particulièrement une séquence de céréale, en particulier une séquence de maïs.

Ladite séquence peut être de manière avantageuse une séquence d'ADNc spécifique de l'état de stress hydrique, isolée à partir d'une lignée de maïs par hybridation différentielle. Une telle séquence est présentée dans le listage de séquence annexé, et désignée SEQ ID N°1. On peut également utiliser une séquence d'ADN génomique codant pour une protéine ASR de maïs, telle que définie par l'une des séquences SEQ ID N°3, SEQ ID N°4 et SEQ ID N°5 ou une séquence homologue à celles-ci, pour autant qu'elle soit exprimée en quantité modifiée par rapport à la quantité habituellement produite par une plante non transformée.

Dans le listage des séquences en annexe,
SEQ ID N° 3 est une séquence d'ADN génomique isolée à partir d'une lignée de maïs qui exprime fortement la protéine ASR,
SEQ ID N°4 est une séquence d'ADN génomique isolée à partir d'une lignée A188 de maïs, témoin, et
SEQ ID N°5 est une séquence d'ADN génomique isolée à partir d'une lignée F₂ de maïs.

Ladite séquence peut coder en particulier pour la séquence d'acides aminés SEQ ID N°2 de la protéine ASR de maïs, ou un variant de celle-ci, par exemple un variant ayant la séquence SEQ ID n° 2 avec une insertion d'un résidu lysine entre les acides aminés 55 et 56 de SEQ ID n° 2.

On peut également utiliser des séquences nucléotidiques codant pour des ASR d'autres végétaux, comme par exemple celles citées précédemment.

L'acide nucléique codant pour une protéine ASR est inséré dans une construction d'acide nucléique, appelée cassette d'expression, et est lié de manière opérante à des éléments permettant son expression et éventuellement sa régulation.

Parmi ces éléments, on peut citer les promoteurs, les activateurs et les terminateurs de transcription.

On peut utiliser préférentiellement un promoteur constitutif tel que le promoteur actine de riz suivi de l'intron actine de riz (PAR-IAR) contenu dans le plasmide pAct1-F4 (Mc Elroy et al., 1991) ou le promoteur 35S (Kay et al., 1987), ou un promoteur tissu spécifique. A titre d'exemple, on peut citer le promoteur HMGW de blé ou encore le promoteur PCRU du gène de la cruciférine de radis, qui permettent tous deux une expression de la protéine d'intérêt dans les graines (Anderson O.D. et al., 1989; Depigny-This et al., 1992). On peut avantageusement utiliser des séquences promotrices induisant l'expression en condition de stress hydrique (Kasuga et al., 1999). Parmi les terminateurs utilisables dans les constructions de l'invention, on peut citer notamment l'extrémité 3' du gène de la nopaline synthétase *d'Agrobacterium tumefaciens* (Depicker et al., 1982). On peut citer également le terminateur polyA 35S du virus de la mosaïque de chou-fleur (CaMV), décrit dans l'article de Franck et al. (1980).

L'expression de la protéine ASR peut encore être régulée par l'utilisation de séquences de type signaux d'adressage peptidiques (chloroplastique, vacuolaire, de rétention endoplasmique...), ou de type séquences introns, séquences enhancers, séquences leaders.

Dans la présente invention, la séquence d'intérêt peut être une séquence nucléotidique codant pour une protéine ASR, ladite séquence étant placée en sens. Lorsque la séquence codant pour une protéine ASR est placée en sens, on obtient une plante transformée qui présente une augmentation de la protéine ASR par rapport à une plante non transformée. Cette plante a l'avantage de résister à un stress hydrique plus efficacement qu'une plante non transformée.

La cassette d'expression est insérée dans un vecteur nucléotidique, tel qu'un plasmide, qui peut en outre comprendre un gène marqueur, par exemple un gène permettant de sélectionner une plante transformée d'une plante qui ne contient pas l'ADN étranger transfecté. Comme gène marqueur, on peut citer un gène conférant une résistance à un antibiotique, par exemple à l'hygromycine (Herrera-Estrella et al., 1983) ou une résistance à un herbicide comme le sulfonamide asulam (WO 98/49316).

Ce vecteur ou toute séquence codant pour une protéine ASR telles que les séquences SEQ ID N°1, SEQ ID N°3, SEQ ID N°4 et SEQ ID N°5 ou séquences homologues à ces dernières, sont utilisables pour la transformation des cellules végétales selon les techniques connues de l'homme de métier, pour l'obtention des plantes présentant une résistance accrue au stress hydrique.

Selon un mode de réalisation du procédé d'invention, les cellules végétales sont transformées par un vecteur tel que défini précédemment, transféré dans un hôte cellulaire susceptible d'infecter lesdites cellules végétales en permettant l'intégration dans le génome de ces dernières, des séquences nucléotidiques d'intérêt initialement contenues dans le génome du vecteur susmentionné. Avantageusement, l'hôte cellulaire utilisé est une souche bactérienne, telle que *Agrobacterium tumefaciens,* notamment selon la méthode décrite dans l'article d'An et al., (1986), ou encore *Agrobacterium rhizogenes,* notamment selon la méthode décrite dans l'article de Guerche et al., (1987).

Par exemple, la transformation des cellules végétales peut être réalisée par le transfert de la région T du plasmide circulaire extra chromosomique indicateur de tumeurs Ti d'*Agrobacterium tumefaciens,* en utilisant un système binaire (Watson et al., 1994). Pour ce faire, deux vecteurs sont construits. Dans l'un de ces vecteurs, la région T a été éliminée par délétion, à l'exception des bordures gauche et droite, un gène marqueur étant inséré entre eux pour permettre la sélection dans les cellules de plantes. L'autre partenaire du système binaire est un plasmide Ti auxiliaire, plasmide modifié qui n'a plus de région T mais qui contient toujours les gènes de virulence *vir,* nécessaire à la transformation de la cellule végétale.

Selon un mode préféré, on peut utiliser la méthode décrite par Ishida et al. (1996), pour la transformation des Monocotylédones.

On peut citer aussi d'autres modes de réalisation du procédé de l'invention, notamment les méthodes de transfert direct de gènes aux cellules végétales telles que la microinjection directes dans des embryoïdes de plante (Neuhaus et al, 1987), l'infiltration sous vide (Bechtold et al. 1993) ou l'électroporation (Chupeau et al, 1989) ou encore la précipitation directe au moyen de PEG (Schocher et al, 1986) ou le bombardement par canon de particules recouvertes de l'ADN plasmidique d'intérêt (Fromm M. et al., 1990).

Selon un autre protocole, la transformation est réalisée selon la méthode décrite par Finer et al. (1992), utilisant le canon à particules de tungstène ou d'or.

L'invention a également pour objet une cellule hôte transformée avec les séquences nucléiques décrites ci-dessus, ainsi qu'une plante ou partie de plante, notamment fruit, semence, grain, pollen, feuille ou tubercule, susceptibles d'être obtenues par l'un des procédés exposés précédemment.

Il peut s'agir par exemple de plantes de grandes cultures (blé, colza, tournesol, pois, soja, orge, en particulier le maïs...) ou de plantes potagères et fleurs.

Les plantes transgéniques hybrides, obtenues par le croisement d'au moins une plante selon l'invention avec une autre, font aussi partie de l'invention.

L'invention concerne notamment une plante présentant une augmentation de l'expression de la protéine ASR par rapport à une plante non transformée, d'un facteur 2 à 3 par exemple. Cette augmentation de l'expression de la protéine ASR confère aux plantes transformées une résistance accrue à un stress hydrique.

La résistance au stress hydrique des plantes transformées selon l'invention, par rapport aux plantes témoins, peut être appréciée à partir de diverses méthodes de mesures morphologiques, physiologiques et/ou biochimiques, pour des conditions d'irrigation particulières. A titre d'exemple, la mesure de la tolérance au stress peut être réalisée par observation phénotypique, (i) de la sénescence foliaire, par des mesures morphologiques et par dosage de la chlorophylle des disques foliaires, (ii) de la protandrie ou date des floraisons des plantes mâles et femelles, (iii) de la croissance de la plante par mesure de la longueur et de la largeur finales des feuilles, ainsi que de la hauteur finale de la plante, et par l'étude de l'enroulement des feuilles, ou encore (iv) du rendement en grain, du poids de mille grains, du nombre d'épis par plante.

Le stress subi par les plantes peut également être évalué par mesure du contenu en ABA (méthode de Quarrie et al, 1988), ou par mesure du potentiel hydrique, ou encore le cas échéant, par le suivi de l'expression de la protéine par électrophorèse bidimensionnelle à partir d'un prélèvement de feuille.

Les plantes obtenues selon l'invention peuvent également être utilisées dans des expériences de complémentation d'allèles, pour la validation de la fonction du gène inséré. L'utilisation des transformants dans des expériences de rétrocroisements permet d'apporter uniquement le gène inséré dans le fond génétique parental, sans autres séquences qui pourraient influer sur le phénotype du recombinant quant à la tolérance à la sécheresse.

De préférence, on couple le gène inséré avec un gène marqueur de sélection, qui facilite le suivi des rétrocroisements et par conséquent le suivi de l'insertion du gène d'intérêt dans la lignée où l'on souhaite valider l'effet.

Le principe consiste à croiser le transformant avec la lignée parentale ne possédant pas l'allèle favorable du gène d'intérêt et comparer les phénotypes de la lignée recombinante avec les lignées parentales. On peut également utiliser des transformants contenant les séquences génomiques dans ces expériences de complémentation. Ce test de complémentation permet de vérifier notamment que la surexpression de l'ADNc en sens complémente l'effet de l'allèle faible (ou nul). Ainsi, on peut par exemple valider que le gène ASR1 est le gène responsable du QTL et du PQL ("Protein quantitative Loci"), trouvés à cette position génétique sur le chromosome 10 chez le maïs. La quantité de cette protéine variant entre différentes lignées, des expressions plus fortes voire des allèles plus favorables peuvent être détectées et exploitées pour l'amélioration des plantes. La détection des plantes présentant des allèles favorables peut être réalisée par des dosages immunologiques avec des anticorps spécifiques de la protéine ASR1 (test ELISA, Western Blot, etc).

Rentre également dans le cadre de l'invention l'utilisation d'un acide nucléique codant pour une ASR ou un fragment de celui-ci, comme sonde ou amorce pour une amplification de type PCR, pour la sélection de plantes, transformées, présentant une meilleure résistance au stress hydrique.

Les séquences d'acides nucléiques codant pour une ASR, telles que celles désignées SEQ ID N°1, SEQ ID N°3, SEQ ID N°4, SEQ ID N°5 ainsi que tout oligonucléotide obtenu à partir de l'une de ces séquences peuvent ainsi être utilisées comme sondes dans des programmes de sélection assistée par marqueur, par exemple pour suivre l'introgression du gène codant pour la protéine ASR de maïs dans une plante. Pour cela au moins une de ces sondes est marquée, par exemple par un isotope radioactif, puis mise en contact avec de l'ADN génomique de la plante, préalablement digéré par des enzymes de restriction, dans des conditions permettant l'hybridation spécifique de la sonde marquée à l'ADN en question. D'autres techniques utilisant par exemple la PCR peuvent également être mises en oeuvre pour faire du génotypage.

Les figures et exemples suivants illustrent l'invention sans en limiter la portée.

### LEGENDE DES FIGURES

La figure 1 représente une carte de restriction du plasmide pWP 280 contenant le promoteur pActin intron-bamase-Nos PolyA.
La figure 2 représente une carte de restriction du vecteur intermédiaire pBIOS 308 contenant ADNc de ZmASR1 en sens.
La figure 3 représente une carte de restriction du vecteur intermédiaire pBIOS 309 contenant ADNc de ZmASR1 en antisens.
La figure 4 représente l'effet de chaque événement de transformation 'antisens' ou 'sens' par rapport à son témoin propre (sensible au Basta), sur la sénescence foliaire, effet mesuré le premier jour de notation après la floraison.
La figure 5 représente une cinétique de l'effet, pour l'ensemble des événements 'sens', 'non transformés' et 'antisens' et placés ou non en condition de stress hydrique, sur la sénescence foliaire.

### EXEMPLES

### EXEMPLE 1 :

### Obtention et clonage de l'ADNc de ZmAsr1 d'une lignée de maïs qui exprime fortement la protéine ASR

### a) Conditions de culture et prélèvements pour les plantes ayant servi pour isoler l'ADNc

Les auteurs de l'invention ont cloné l'ADNc de ZmAsr1 à partir de lignées de maïs Io (Riccardi et al, 1998) ou de lignées de maïs sélectionnées selon les mêmes critères que Io.

Les plantes de maïs sont cultivées dans de la perlite en conditions contrôlées dans une chambre de culture (éclairement : 450 mmol m⁻² s⁻¹, photopériode : 16h, température jour/nuit : 25°C/20°C, humidité relative 60%), arrosée avec une solution nutritive. Lorsque les plantes ont atteint le stade de «5 feuilles» (5^{ème} feuille émergente), soit l'arrosage est arrêté pour les plantes en conditions de stress hydrique, soit l'arrosage se poursuit pour les plantes témoins. 10 jours plus tard, on procède à des prélèvements sur la partie engainée du limbe de la 7^{ème} feuille.

### b) Isolement des ADNc spécifiques de l'état de stress hydrique par hybridation différentielle

Une banque d'ADNc est préparée à partir d'ARNm de feuilles de plantes stressées et du kit de clonage Lambda Zapll cDNA synthesis/Gigapack Goldl (Stratagene, La Jolla, USA), selon les indications du fabricant.

Les ARNm préparés à partir des feuilles de plantes stressées et des plantes témoins sont transcrits en ADNc radiomarqué en utilisant 100 µCi de ³²P-dATP et des hexanucléotides servant d'amorces aléatoires (Sambrook et al., 1989). Les ADNc simple brin, provenant soit de la plante stressée soit de la plante témoin, sont mis à hybrider dans une même proportion sur les clones d'ADNc de la banque. La température d'hybridation dans le système tampon phosphate/SDS/EDTA (Church et Gilbert, 1984) est de 68°C et les lavages finaux sont effectués avec des solutions renfermant 0,1xSSC 0,05% (poids/volume) SDS.

Les clones marqués sont récupérés par excision in vivo du phagemide selon le protocole Stratagene utilisant E.coli SOLR et le phage helper 'Exassist'. L'ADN de ces clones est séquencé et comparé à des banques de séquences d'acides nucléiques (BLAST) selon la méthode décrite dans Altschul et al. (1990). Un de ces clones présente une forte homologie avec Asr1 de tomate et est dénommé ZmAsr1 pour Zea maize Asr1.

### EXEMPLE 2 :

### Séquences génomiques de ZmASR1

Les amorces cASR1-1F (5'-TGTCGATCCAATTGTCACTT-3')= SEQ ID N°6 et cASR1-740R (5'-TGGAGAAACGTAAACAACTA-3')= SEQ ID N°7, définies aux deux extrémités de la séquence d'ADNc de la protéine ASR1, sont utilisées en amplification PCR sur l'ADN total de lignée de maïs. Les réactions PCR sont réalisées selon les techniques classiques.

Les produits PCR sont analysés en électrophorèse: une bande à 900 pb est récupérée pour en extraire l'ADN et le cloner selon les techniques classiques. Après vérification de leur taille, les inserts sont extraits et séquencés.

### EXEMPLE 3 :

### Construction de gènes chimériques permettant l'expression constitutive de la protéine ASR1 ou bien d'un antisens conduisant à son inhibition

Dans un premier temps est opérée la construction de 2 vecteurs plasmidiques de base pBIOS 306 et pBIOS 307 contenant le promoteur actine-intron actine (pAct), l'ADNc du gène Asr1 respectivement en sens et antisens et le terminateur de la nopaline synthétase (terNos) qui amène un signal de polyadénylation fonctionnel chez de nombreuses espèces végétales.

Des vecteurs intermédiaires sont ensuite réalisés pour la recombinaison homologue avec le vecteur pSB1 de Japan Tobacco (EP 672 752) dans *Agrobacterium tumefaciens* souche LBA 4404 (Hoekema *et al,* 1983).

Le transfert suivi de l'expression des gènes (gène de sélection et gène d'intéret) dans le maïs est basée sur les propriétés naturelles d'*Agrobacterium tumefaciens* (Zambrisky et al. 1989) et sur la stratégie du plasmide superbinaire (Hiei *et al;* 1994 et Ishida *et al;* 1996).

Les enzymes de restriction utilisées pour les clonages sont fournies par New England Biolabs (New England Biolabs, UK). Les réactions enzymatiques sont réalisées en suivant les protocoles décrits, par Sambrook et *al.,* dans le manuel Molecular cloning (Molecular Cloning : A Laboratory Manual, 1989, Cold Spring Harbor Laboratory Press, Cold Spring Harbor, New York.

### a) Construction des vecteurs plasmidiques de base pour l'expression constitutive du gène Asr1 et de son antisens

Les construits moléculaires permettant l'expression du gène Asr1 en sens et antisens de façon constitutive ont été réalisés comme décrit ci-après :
- clonage du fragment I/Xhol de 795 pb (ADNc Asr1 = SEQ ID n°1) dans le vecteur pBIOS 298 restreint à EcoRV.

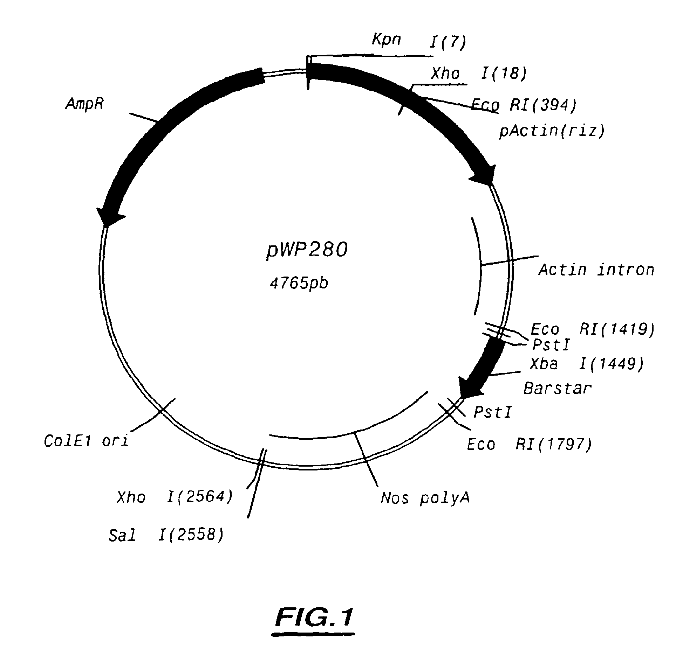

Le vecteur pBIOS 298 contient le promoteur actine-intron actine (pAct) (Mc Elroy et al. 1991) et le terminateur Nos. Ce vecteur a été généré par délétion du fragment PstI de 366 pb (gène Barstar) du vecteur pWP 280, contenant la cassette pActin-intron -Barstar-Nos polyA (Figure 1).

Ce clonage non orienté permet d'obtenir 2 nouveaux vecteurs:
- le vecteur pBIOS 306 porteur du gène pAct-ZmAsr1 sens-terNos
- le vecteur pBIOS 307 porteur du gène pAct-ZmAsr1 antisens-terNos

L'orientation de l'ADNc par rapport au promoteur actine a été déterminée par restriction enzymatique simple avec Eagl et double restriction enzymatique Hind III - EcoRV.

### b) Construction des vecteurs intermédiaires pour la recombinaison homologue avec pSB1 (obtention de plasmides superbinaires)

Les vecteurs servant à la recombinaison homologue dans *Agrobacterium tumefaciens* sont dérivés du vecteur pBIOS 273.

### * Construction du plasmide pBIOS 273

Le vecteur de base pour la recombinaison homologue est le vecteur pBIOS 273. Ce vecteur a été généré en 2 étapes :
- clonage du fragment BspDI/XhoI (pAct-Bar-terNos) du vecteur pDM 302 (Cao et al. 1992) dans les sites Smal et BspDI du vecteur pSB12 (Japan Tobacco). Le vecteur résultant de ce clonage est appelé pBIOS 272.
- délétion du site XhoI en position 3363 du vecteur pBIOS 272 par digestion partielle avec XhoI et action de l'ADN Polymerase I, large (Klenow) fragment. Le vecteur obtenu, possédant un site unique XhoI, est nommé pBIOS 273.

### * Génération des vecteurs intermédiaires de recombinaison contenant l'ADNc Asr1 en sens ou en antisens

Ces construits ont été générés à partir du vecteur pBIOS 274, dérivé du vecteur pBIOS 273 par clonage du fragment (ProA9-Barnase-terCaMV) XhoI du vecteur pWP 128 (Paul et al. 1992) dans le vecteur pBIOS 273 restreint à XhoI.

Les vecteurs intermédiaires pBIOS308 et pBIOS309 ont été obtenus par clonage des fragments Sall/XhoI de 2970 pb des vecteurs pBIOS 306 et pBIOS 307 dans les sites BspDl/Xho I du vecteur pBIOS 274.

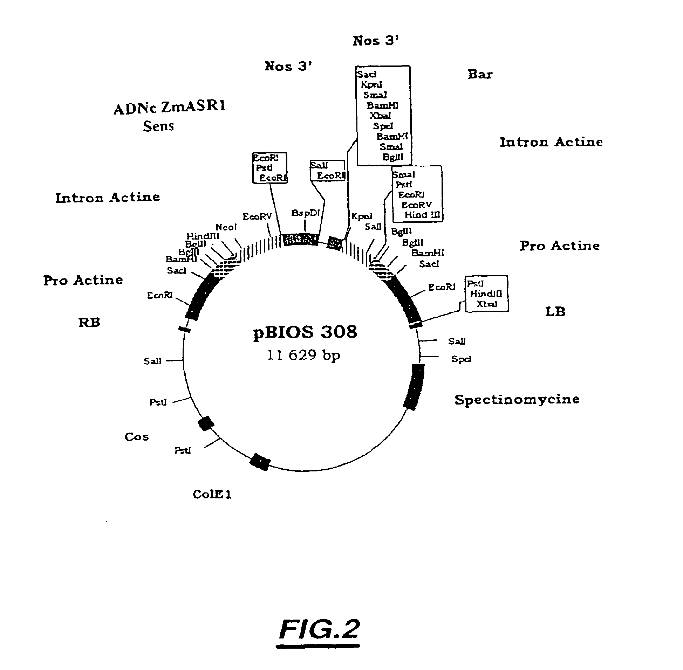

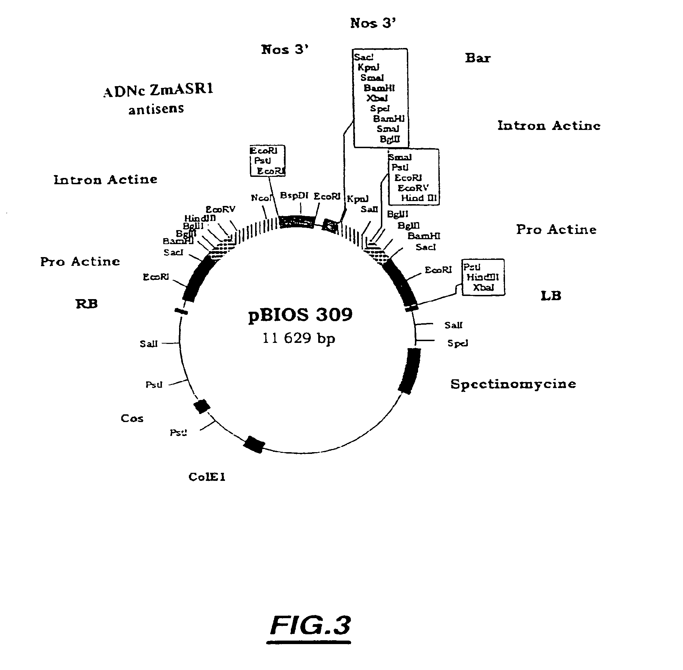

Ces clonages permettent ainsi de substituer le gène pA9-Barnase-terCaMV du vecteur pBIOS 274 par le gène pAct-ZmAsr1 sens-terNos, vecteur résultant appelé pBIOS 308 (Figure 2) ou par le gène pAct-ZmAsr1 antisens-terNos, vecteur résultant appelé pBIOS 309 (Figure 3).

### c) Construction des vecteurs superbinaires de transformation pour l'expression du gène Asr1 et de son antisens dans Agrobacterium tumefaciens et dans les plantes de maïs

### * Construction des vecteurs superbinaires

Les vecteurs utilisés pour la transformation du maïs sont issus de la recombinaison homologue des plasmides pBIOS 308 et pBIOS 309 avec le vecteur pSB1 (EP 672 752). Le vecteur pSB1 contient les gènes virB et virG du plasmide Ti pTiBo542 présent dans la souche d'*Agrobacterium tumefaciens* A281 (ATCC 37349), le gène de résistance à la tétracycline, une origine de réplication fonctionnelle chez *E. coli* et *Agrobacterium* et une région homologue retrouvée dans les vecteurs intermédiaires pBIOS 308 et pBIOS 309. La présence de cette région homologue dans le plasmide receveur (pSB1) et les plasmides intermédiaires (pBIOS 308 et pBIOS 309) est à l'origine du phénomène de recombinaison homologue.

Les vecteurs intermédiaires pBIOS 308 et pBIOS 309 sont introduits dans les cellules d'*Agrobacterium tumefaciens* contenant le vecteur pSB1 par électroporation en utilisant le GIBCO BRL CELL PORATOR Voltage Booster selon la méthode décrite par Mattanovitch *et al*.(1989) et le protocole donné par le fournisseur (Life Technologies, USA).

Les agrobactéries contenant les vecteurs superbinaires sont sélectionnées sur milieu YT CaCl₂ en présence de rifampicine et de spectinomycine à une concentration de 50mg/l. Le gène de résistance à la rifampicine est porté par le chromosome bactérien. La résistance à la spectinomycine, portée par les plasmides pBIOS 308 et pBIOS 309 (origine de réplication dans *E. coli*), ne pourra s'exprimer qu'après recombinaison homologue avec le vecteur pSB1 (origine de réplication fonctionnelle chez *Agrobacterium* et *E. coli*).

Les plasmides superbinaires obtenus après recombinaison sont nommés pRec 308 (pBIOS 308 x pSB1) et pRec 309 (pBIOS 309 x pSB1). Ils possèdent des origines de réplication fonctionnelles à la fois dans *E. coli* et *Agrobacterium tumefaciens,* les gènes de résistance à la tétracycline et à la spectinomycine, le T-DNA dans lequel se trouvent les cassettes d'expression des gènes Bar et Asr1 (ADNc sens ou antisens) et les gènes de virulence virB et virG du plasmide pTiBo542.

### * Caractérisation des vecteurs superbinaires pRec 308 et pRec 309

Ces plasmides superbinaires pRec 308 (gène Asr1 en sens) et pRec 309 (gène Asr1 en antisens) sont caractérisés par restriction enzymatique avec SalI. Une analyse Southern des fragments de restriction SalI est ensuite effectuée avec la sonde Bar et la sonde du gène Asr1 (cette sonde correspond au fragment EcoRI/XhoI de 795 pb du vecteur pHHU516 donc à l'ADNc complet). Les profils obtenus sont ceux attendus.

### EXEMPLE 4 :

### Transformation des plantes de maïs

La transformation des plantes de maïs est réalisée selon le protocole d'Ishida et al. (1996)

La transformation débute avec une co-culture où les embryons immatures des plantes de maïs (taille variant de 1 à 1,2 mm) sont mis en contact pendant 5 minutes avec *Agrobacterium tumefaciens* LBA 4404 contenant les vecteurs superbinaires pRec 308 ou pRec 309. Les embryons sont ensuite placés sur milieu LSAs pendant 3 jours à l'obscurité et à 25°C.

L'étape suivante est celle de la première sélection des cals transformés : les "embryon-cals" sont transférés sur milieu LSD 5 contenant de la phosphinotricine à 5mg/l et de la céfotaxime à 250 mg/l (Elimination d'*Agrobacterium tumefaciens*). Cette étape est menée 2 semaines à l'obscurité et à 25°C.

La deuxième étape de sélection s'effectue par transfert des embryons qui se sont développés sur milieu LSD 5 sur milieu LSD 10 (phosphinotricine à 10 mg/l) en présence de céfotaxime, pendant 3 semaines dans les mêmes conditions qu'en première sélection (25°C, obscurité).

La troisième étape de sélection consiste à exciser les cals de type I (fragments de 1 à 2 mm) et à les transférer pendant 3 semaines à l'obscurité et à 25°C sur milieu LSD 10 en présence de céfotaxime.

La régénération des plantules est ensuite effectuée en excisant les cals de type I qui ont proliféré et en les transférant sur milieu LSZ en présence de phosphinotricine à 5 mg/l et de céfotaxime pendant deux semaines à 22°C et sous lumière continue. Les plantules ayant régénéré sont transférées sur milieu d'enracinement (Ishida et al, 1996) pendant deux semaines à 22°C et sous illumination continue pour l'étape de développement.

Les plantes obtenues sont alors transférées au phytotron en vue de leur acclimatation.

### EXEMPLE 5:

### Mise en évidence de l'expression de la protéine ZmASR1 dans les plantes transformées

Les protéines des échantillons de feuilles provenant des plantes transformées ont été extraites selon la méthode de Damerval et al (1986), puis ont été analysées par électrophorèse bidimensionnelle (EBD) suivant le protocole de Riccardi et al. (1998).

L'électrophorèse bidimensionnelle consiste à séparer les polypeptides en fonction de leur point isoélectrique et en fonction de leur poids moléculaire (électrophorèse en présence de SDS). Avant électrophorèse, les protéines sont extraites et maintenues dans des conditions dénaturées : la structure quaternaire est éliminée. Les différents polypeptides constituant les protéines oligomériques migrent indépendamment durant les deux électrophorèses. Les gels sont ensuite colorés au nitrate d'argent.

Le gel bidimensionnel ainsi obtenu est comparé à celui réalisé à partir de protéines de feuilles de plantes A188 non transformées.

Les résultats obtenus des plantes transformées avec la séquence codante placée en « sens » montrent, par simple examen visuel de ces deux gels, une expression plus intense de la protéine ASR1 dans les plantes transformées par rapport aux plantes témoins A188.

Le spot ASR observé sur les plantes transformées avec le construit antisens semble être toujours détectable, mais avec une intensité plus faible, ce qui montre que les transformants 'antisens' expriment moins la protéine que les plantes témoins.

Cette analyse protéique met donc en évidence une expression de la protéine ASR1 dans les plantes transformées, en quantité modifiée par rapport aux plantes non transformées.

### EXEMPLE 6 :

### Mesure de la tolérance au stress hydrique des plantes transgéniques obtenues selon l'invention

La résistance au stress hydrique des plantes transformées selon l'invention, par rapport aux plantes témoins, peut être appréciée à partir de diverses méthodes d'analyses phénotypique, physiologique et/ou biochimique, pour des conditions d'irrigation particulières- en conditions normales (culture traditionnelle avec arrosage) et en conditions de stress hydrique.

A titre d'exemple, les conditions hydriques en champ peuvent être les suivantes :
- une irrigation normale dans la partie irriguée sur la base de 5 mm par jour pilotée par des tensiomètres placés à 30,50,70 cm de profondeur.
- une irrigation restrictive, sans apport d'eau si possible jusqu'à 10 jours après la floraison. A cette date approximative, il est décidé d'apporter de l'eau lorsque le stress est jugé trop intense, le rythme d'apport ne devra pas excéder 3mm par jour.

Les données météo et les conditions d'irrigation sont enregistrées.

Les notations effectuées aux différentes périodes de floraison et de récolte consistent à mesurer :

### a) Mesure de la tolérance au stress par observation phénotypique

Les analyses génétiques effectuées antérieurement ont suggéré un rôle potentiel de ASR1 dans la sénescence foliaire et la protandrie (décalage entre la floraison mâle et femelle) en conditions de sécheresse. Ces caractères sont donc étudiés préférentiellement.

La sénescence foliaire peut être étudiée par des mesures morphologiques qui consistent à compter le nombre de feuilles desséchées et vertes à 4 dates espacées de 15 jours, à partir de la date de floraison ou à différents stades de développement. Lorsque des comportements très différents sont observés concernant l'enroulement et la couleur des feuilles, une mesure est réalisée suivant une échelle de 0 à 5 (de la plus tolérante à la moins tolérante au stress).

La sénescence peut également être mesurée par dosage de la chlorophylle sur des prélèvements de disques foliaires dans la feuille de l'épi à la floraison.

La protandrie ou décalage entre les dates de floraisons des plantes mâles (présence de pollen) et femelles (sortie des soies) est mesurée comme suit : les dates d'apparition des soies et du pollen sont notées individuellement pour chaque plante, et la dynamique est représentée par la courbe du pourcentage de plantes ayant fleuri en fonction du temps.

Par ailleurs, différentes mesures sont effectuées à la récolte pour évaluer l'effet de la tolérance au stress sur la production en grain, notamment : le pourcentage de fécondation (rapport du nombre de grains par épi / nombre d'ovules fécondables), le nombre de rangs par épi, le nombre de grains par rang, l'humidité des grains, le poids de 1000 grains, le nombre de plantes fusariées.

L'utilisation d'un test d'identification non destructif utilisant le basta permet de distinguer facilement dans chaque descendance transgénique les plantes qui sont résistantes au basta des plantes sensibles, les plantes résistantes contenant le gène Asr lié génétiquement au gène marqueur de sélection, sauf événement de recombinaison. Ce test consiste à badigeonner l'extrémité d'une feuille avec une solution de basta et observer le phénotype résultant, sans que la vitalité de la plante entière ne soit remise en cause : l'extrémité de la feuille se nécrose et dessèche lorsque la plante est sensible, ou reste verte si la plante est résistante. Ceci permet d'observer sur les mesures de tolérance au stress si le transgène ASR influence positivement la réponse au stress en fonction de l'expression du gène en sens ou en anti-sens.

### b) Evaluation du stress subi par les plantes

Des prélèvements sont effectués sur les feuilles, afin de mesurer le contenu en ABA (méthode de Quarrie et al, 1988), et le cas échéant, l'expression de la protéine par électrophorèse bidimensionnelle.

Le stress subi par les plantes peut être évalué par des mesures du potentiel hydrique de base à l'aide d'une chambre à pression portable sur des plantes non transformées, en conditions témoin et en conditions de stress hydrique. Des mesures de la teneur relative en eau des feuilles peuvent également être réalisées.

La sénescence foliaire, qui permet une réduction de la surface d'évaporation et une re-mobilisation des métabolites vers le reste de la plante, a été mesurée comme décrit ci-dessus, sur des plantes adultes après floraison, par comptage du nombre de feuilles dont au moins 50% de la surface est sèche.

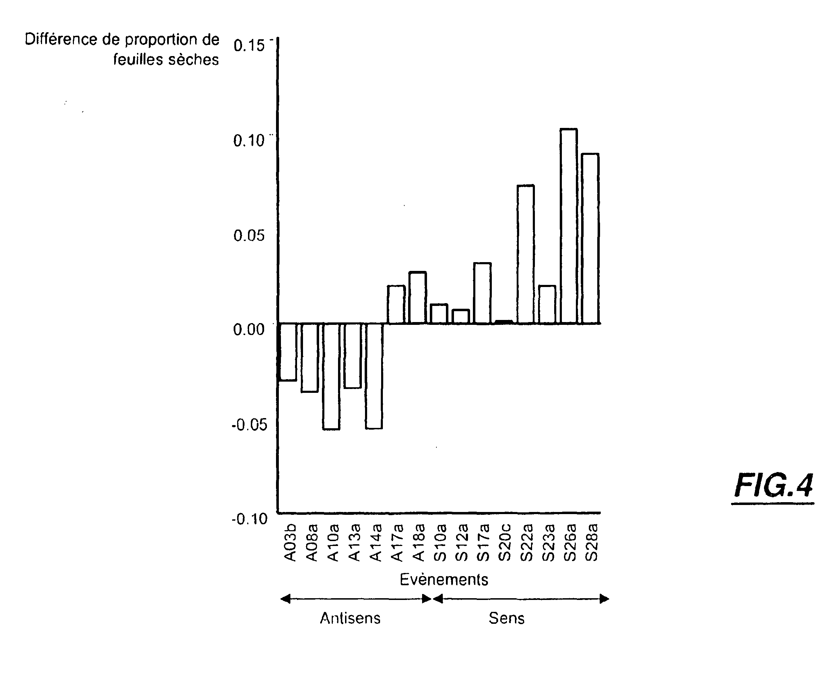

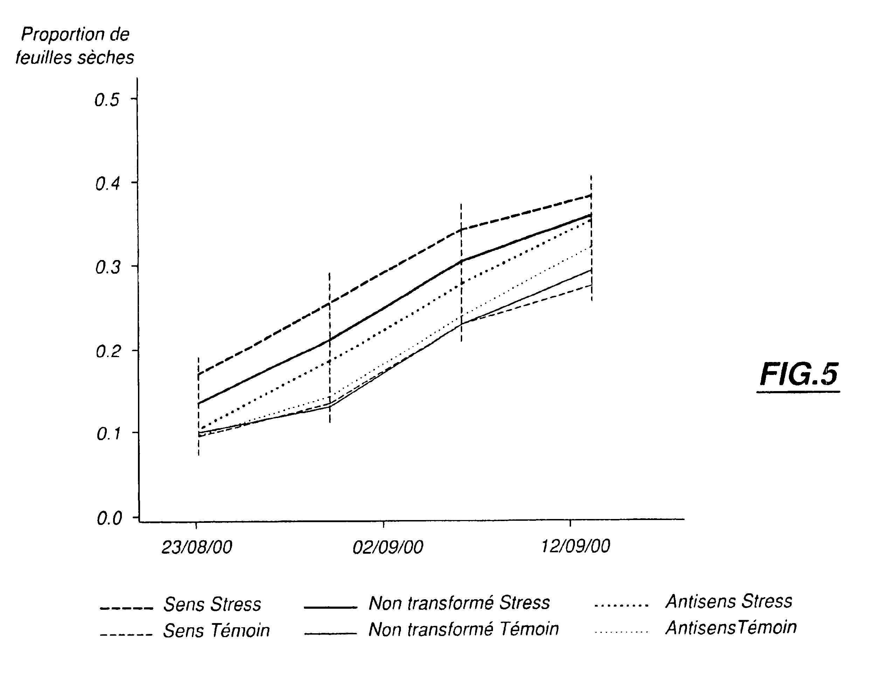

On observe un effet significatif de l'expression de la protéine ASR sur la proportion de feuilles sèches. En effet, rapportés à leurs témoins propres, les événements 'sens' présentent, à un temps donné, une proportion de feuilles sèches plus grande que des événements 'antisens' (Figure 4). De même, en condition de stress hydrique, les mesures de cinétique de sénescence foliaire montrent que les événements 'sens' sénescent plus vite que les 'non transformés' et que les événements 'antisens' sénescent moins vite que les 'non transformés' (Figure 5).

Par événement 'sens', on entend un événement issu d'une étape initiale de transformation avec une cassette d'expression contenant la séquence ASR placée en sens. Par événement 'antisens', on entend un événement issu d'une étape initiale de transformation avec une cassette contenant la séquence ASR placée en antisens.

Les événements 'sens', qui sénescent plus vite que les 'non transformés' en condition de stress hydrique, présentent donc un avantage sélectif de tolérance au stress hydrique, notamment pour le cas d'un stress de longue durée (réduction de la surface d'évaporation et re-mobilisation des métabolites vers le reste de la plante).

Les événements 'antisens', qui sénescent moins vite que les 'non transformés' en condition de stress hydrique, présentent également un intérêt, en particulier dans le cas d'un déficit hydrique de courte durée. En effet, ces plantes auront conservé une plus grande surface d'évaporation et bénéficieront donc plus pleinement d'un apport d'eau (pluies ou irrigation) ultérieur.

D'autre part, les mesures du stress subi par les plantes (contenu des feuilles en ABA) ont révélé un stress léger mais hautement significatif: respectivement 616 et 512 ng ABA/g matière sèche dans les plantes stressées et témoins, soit une augmentation d'environ 100 ng d'ABA par gramme de matière sèche dans les plantes stressées. Cette réponse est la même pour les événements 'sens' et 'antisens', ainsi que pour les plantes non transformées. La transformation ne semble donc pas avoir d'effet sur l'accumulation de l'ABA dans les feuilles.

Ces résultats confirment donc qu'en présence d'un stress hydrique léger et d'une expression ASR faible dans les plantes transformées, des différences significatives sont déjà observées.

Par ailleurs, l'effet de la tolérance au stress hydrique sur la production en grains a été mesuré par rapport au rendement en grain, du poids de milles grains et du nombre d'épis par plante. Les résultats obtenus, avec un stress hydrique faible, montrent des mesures de rendement en grain comparables entre des plantes transformées ('sens' et 'antisens') et des plantes non transformées, prises en condition de stress ou en condition normale.

La transformation d'une part et la tolérance au stress hydrique d'autre part ne semblent donc pas affecter le rendement en grain des plantes.

Des mesures de croissance des plantes ont également été effectuées. Les longueurs de 3 feuilles en dessous et au-dessus de l'épi ont été mesurées sur des plantes 'sens' et 'antisens' après floraison, en l'absence de stress hydrique. Une différence hautement significative a été observée pour les 3 feuilles :

| | | | | |
|---|---|---|---|---|
| pour les feuilles | F0, | antisens | = 78,41 cm | (p<0,01) |
| | | sens | = 76,11 cm | |
| | F1, | antisens | = 77,96 cm | (p<0,01) |
| | | sens | = 75,76 cm | |
| | F2, | antisens | = 75,04 cm | (p<0,01) |
| | | sens | = 72,94 cm | |

Globalement, on observe donc une différence significative de 2 cm de longueur pour ces 3 feuilles, les feuilles des plantes 'sens' étant plus petites que celles des 'antisens'. Cette diminution de la croissance de la feuille observée chez les événements 'sens' corrèle donc avec une plus grande sénescence, les deux phénomènes aboutissant à une surface d'évaporation réduite, qui permet à la plante de mieux tolérer le stress hydrique.

### BIBLIOGRAPHIE

- An et al. (1986), Plant Physiol. 81 : 86-91
- Altschul et al. (1990), J. Mol. Biol. 215:403-410
- Anderson O.D. et al., (1989),T.A.G., 77:689-700
- Bechtold et al. (1993), Comptes rendus Académie des Sciences Paris Serie 3,316:1194-1199
- Canel C. et al. (1995), Plant Physiol. 108 (1995), 1323-1325.
- Chupeau et al. (1989), Biotechnology, 7(5):503-508
- Church et Gilbert, (1984) Proc. Natl. Acad. Sci. USA, 81: 1991-1995
- Damerval et al. Electrophoresis 7:52-54, 1986
- Depicker et al., (1982), J. Mol. Appl. Genet., 1, 561-573.
- Depigny-This et al., (1992), Plant Mol. Biol., 20:467-479
- De Vienne et al., (1999), Journal of Experimental Botany, 50(332) : 303-309.
- Finer et al. (1992). Plant Cell Report, 11:323-328
- Franck et al. (1980), Cell, 21:285-294
- Fromm M. et al. (1990), Biotechnology, 8:833-839
- Guerche et al. (1987), Mol. Gen. Genet., 206:382
- Herrera-Estrella et al. (1983), EMBO J.2,987-995
- Hiei et al. (1994). The Plant Journal, 6, 271-282.
- Hoekema et al. (1983). Nature, 303, 179-180.
- lusem et al., (1993). Plant Physiol., 102:1353-1354
- Ishida et al.(1996), Nature Biotechnology 14 : 754-750
- Kasuga et al. (1999), Nature Biotechnologie, 17:287-291
- Kay et al., (1987) Science, 236:4805
- Mattanovitch, et al., (1989), Nucleic Acids Research, 17(16):6747.
- Mc Elroy et al. (1991). Molecular and General Genetics, 231(1), 150-160.
- Neuhaus et al. (1987), Theoretical and applied Genet., 75(1):30-36
- Quarrie et al. (1988),Planta 173 :330-339
- Riccardi et al. (1998). Plant Physiol., 117:1253-1263,
- Sambrook et al. (1989). Molecular cloning, A laboratory manual, 2^{nd} Edition cold spring harbor laboratory press
- Schocher et al. (1986). Biotechnology 4:1093-1096
- Silhavy D. et al. (1995), Plant. Mol. Biol. 27 (1995), 587-595.
- Thomas et al., (1999), Plant Science 140, 21-30
- Watson et al. (1994), ADN recombinant, Ed. De Boeck Université pp 273-292
- Zambryski et al. (1989) Cell, 56, 193-201.
- Zeewart et Creelman (1988), Annu. Rev. Plant Physiol. Plant Mol. Biol 39 : 439-473

## Revendications

1. Procédé d'obtention d'une plante présentant une quantité modifiée de protéine ASR, lui conférant une meilleure résistance au stress hydrique par rapport à une plante non transformée, comprenant les étapes consistant à :
- transformer au moins une cellule de plante avec un vecteur, contenant une cassette d'expression comprenant une séquence nucléotidique codant pour une protéine ASR qui est exprimée naturellement par une plante en réponse à un stress hydrique et dont la séquence d'acides aminés présente au moins 50% de similitude avec SEQ ID n° 2, ladite séquence nucléotidique étant insérée en sens;
- cultiver la cellule ainsi transformée de manière à générer une plante contenant dans son génome ladite cassette d'expression.

2. Procédé selon la revendication 1, dans lequel ladite protéine ASR est issue d'une céréale, telle que le maïs.

3. Procédé selon la revendication 2, dans lequel ladite protéine ASR comprend la séquence SEQ ID N°2.

4. Procédé selon l'une des revendications 1 à 3, dans lequel la cassette d'expression comprend un promoteur permettant l'expression constitutive de la séquence codant pour l'ASR, par exemple le promoteur actine-intron.

5. Procédé selon l'une des revendications 1 à 3, dans lequel la cassette d'expression comprend un promoteur induisant l'expression en condition de stress hydrique.

6. Procédé selon l'une des revendications 1 à 5, dans lequel la cassette d'expression contient une séquence choisie parmi SEQ ID n° 1, n° 3, n° 4 ou n° 5.

7. Plante ou partie de plante, susceptible d'être obtenue par le procédé selon l'une quelconque des revendications 1 à 8.

8. Plante ou partie de plante selon la revendication 7, présentant une augmentation de l'expression de la protéine ASR par rapport à une plante non transformée.

9. Plante ou partie de plante selon la revendication 7 ou 8, **caractérisée en ce qu'**il s'agit d'une plante de grande culture choisie parmi le maïs, le blé, le colza, le tournesol et le pois, en particulier le maïs.

10. Plante transgénique hybride susceptible d'être obtenue par un procédé comprenant :
a) l'obtention de deux plantes transgéniques selon le procédé de l'une quelconque des revendications 1 à 8, et
b) le croisement de ces plantes.

11. Utilisation d'un acide nucléique codant pour une protéine ASR qui est exprimée naturellement par une plante en réponse à un stress hydrique et dont la séquence d'acides aminés présente au moins 50% de similitude avec SEQ ID n° 2, pour l'obtention d'une plante transgénique présentant une quantité modifiée de protéine ASR, lui conférant une résistance accrue au stress hydrique par rapport à une plante non transformée.

12. Utilisation d'un acide nucléique codant pour une protéine ASR qui est exprimée naturellement par une plante en réponse à un stress hydrique et dont la séquence d'acides aminés présente au moins 50% de similitude avec SEQ ID n° 2, ou un fragment de celui-ci, comme sonde ou amorce pour amplification pour la sélection de plantes transformées selon l'une des revendications 7 à 10, présentant une meilleure résistance au stress hydrique.

## Claims

1. Procedure for obtaining a plant which possesses modified quantities of ASR protein which confer on it improved resistance to water deficit stress in relation to non-transformed plants, and which includes the following steps which involve:
- transforming at least one plant cell using a vector which contains an expression cassette which includes an ASR protein coding nucleotide sequence which is naturally expressed by a plant in response to water deficit stress and whose amino acid sequence exhibits at least a 50% similarity with SEQ ID no. 2, with the said nucleotide sequence being directionally inserted.
- cultivating the cell that has been thus transformed in order to generate a plant which contains the said expression cassette in its genome.

2. Process according to claim 1, in which the said ASR protein is produced by a cereal, such as maize.

3. Process according to claim 2, in which the said ASR protein includes the sequence SEQ ID no.2.

4. Process according to any of claims 1 to 3, in which the expression cassette includes a promoter which allows constitutive expression of the coding sequence for the ASR, for example the actin-intron promoter.

5. Process according to any of claims 1 to 3, in which the expression cassette includes a promoter which induces the expression under conditions of water deficit stress.

6. Process according to any of claims 1 to 5, in which the expression cassette includes a sequence which is chosen from amongst SEQ ID no.1, no.3, no.4 or no.5.

7. Plant or part of plant which can be obtained by the process according to any of claims 1 to 6 whatsoever.

8. Plant of part of plant according to claim 7 which exhibits an increase in expression of the ASR protein in relation to a non-transformed plant.

9. Plant or part of a plant according to claim 7 or 8, **characterised by** the fact that it is a large-scale farming plant selected from amongst maize, wheat, rape, sunflower and peas, and in particular maize.

10. Transgenic hybrid plant which can be obtained by a process which involves:
a) obtaining two transgenic plants according to the process in any of claims 1 to 6 whatsoever, and
b) crossing these plants.

11. The use of an ASR-protein coding nucleic acid which is naturally expressed by a plant in response to water deficit stress and whose amino acid sequence exhibits at least 50% similarity with SEQ ID no.2, in order to obtain a transgenic plant which possesses modified quantities of ASR protein, which confers upon it increased resistance to water deficit stress in relation to non-transformed plants.

12. The use of an ASR-protein coding nucleic acid which is naturally expressed by a plant in response to water deficit stress and whose amino acid sequence exhibits at least 50% similarity with SEQ ID no.2, or a fragment of this, as a probe or primer for amplification for the selection of plants transformed according to one of claims 7 to 10 which offer better resistance to water deficit stress.

## Patentansprüche

1. Verfahren zur Herstellung einer Pflanze, die eine modifizierte Menge an dem Protein ASR aufweist, die ihr eine bessere Resistenz gegenüber Trockenstress im Vergleich mit einer nicht transformierten Pflanze verleiht, das die Stufen umfasst, die darin bestehen:
- mindestens eine Pflanzenzelle mit einem Vektor zu transformieren, der eine Expressionskassette enthält, die eine Nucleotidsequenz umfasst, die für ein Protein ASR codiert, das natürlicherweise von einer Pflanze als Antwort auf Trockenstress exprimiert wird, und dessen Aminosäuresequenz mindestens 50 % Ähnlichkeit mit der SEQ ID Nr. 2 besitzt, wobei die Nucleotidsequenz in *sense* insertiert wird, und
- die so transformierte Zelle derart zu kultivieren, dass eine Pflanze erzeugt wird, die in ihrem Genom diese Expressionskassette enthält.

2. Verfahren nach Anspruch 1, in welchem das Protein ASR von einem Getreide wie Mais stammt.

3. Verfahren nach Anspruch 2, in welchem das Protein ASR die Sequenz SEQ ID Nr. 2 umfasst.

4. Verfahren nach einem der Ansprüche 1 bis 3, in welchem die Expressionskassette einen Promotor umfasst, der die konstitutive Expression der Sequenz erlaubt, die für ASR codiert, beispielsweise den Aktin-Intron-Promotor.

5. Verfahren nach einem der Ansprüche 1 bis 3, in welchem die Expressionskassette einen Promotor umfasst, der die Expression unter Trockenstressbedingungen induziert.

6. Verfahren nach einem der Ansprüche 1 bis 5, in welchem die Expressionskassette eine Sequenz enthält, die aus SEQ ID Nr. 1, Nr. 3, Nr. 4 oder Nr. 5 ausgewählt ist.

7. Pflanze oder Pflanzenteil, die (das) durch das Verfahren nach einem der Ansprüche 1 bis 6 erhalten werden kann.

8. Pflanze oder Pflanzenteil nach Anspruch 7, die (das) eine Erhöhung der Expression des Proteins ASR gegenüber einer nicht transformierten Pflanze aufweist.

9. Pflanze oder Pflanzenteil nach Anspruch 7 oder 8, **dadurch gekennzeichnet, dass** es sich um eine landwirtschaftliche Kulturpflanze handelt, die aus Mais, Getreide, Raps, Sonnenblumen und Erbsen, insbesondere Mais, ausgewählt ist.

10. Hybride transgene Pflanze, die durch ein Verfahren hergestellt werden kann, das:
a) das Erhalten von zwei transgenen Pflanzen gemäß dem Verfahren nach einem der Ansprüche 1 bis 6 und
b) die Kreuzung dieser Pflanzen
umfasst.

11. Verwendung einer Nucleinsäure, die für ein Protein ASR codiert, das natürlicherweise von einer Pflanze als Antwort auf Trockenstress exprimiert wird, und dessen Aminosäuresequenz mindestens 50 % Ähnlichkeit mit der SEQ ID Nr. 2 besitzt, zur Herstellung einer transgenen Pflanze, die eine modifizierte Menge an Protein ASR aufweist, die ihr eine erhöhte Resistenz gegenüber Trockenstress im Vergleich mit einer nicht transformierten Pflanze verleiht.

12. Verwendung einer Nucleinsäure, die für ein Protein ASR codiert, das natürlicherweise von einer Pflanze als Antwort auf Trockenstress exprimiert wird, und dessen Aminosäuresequenz mindestens 50 % Ähnlichkeit mit der SEQ ID Nr. 2 besitzt, oder eines Fragments davon als Sonde oder Primer zur Amplifikation für die Auswahl transformierter Pflanzen nach einem der Ansprüche 7 bis 10, die eine bessere Resistenz gegenüber Trockenstress besitzen.
